## Europäisches Patentamt
## European Patent Office
## Office européen des brevets

(19)

(11) Numéro de publication: **0 119 916**
**B1**

(12) **FASCICULE DE BREVET EUROPÉEN**

(45) Date de publication du fascicule du brevet:
**10.08.88**

(51) Int. Cl.⁴: **A 61 F 13/00,** A 61 G 13/00

(21) Numéro de dépôt: **84400505.8**

(22) Date de dépôt: **13.03.84**

(54) **Isolateur local pour la cicatrisation d'une plaie en milieu aseptique.**

(30) Priorité: **18.03.83 FR 8304470**

(43) Date de publication de la demande:
**26.09.84 Bulletin 84/39**

(45) Mention de la délivrance du brevet:
**10.08.88 Bulletin 88/32**

(84) Etats contractants désignés:
**BE CH DE GB IT LI**

(56) Documents cité:
**DE-A-2 936 256**
**FR-A-2 188 437**
**GB-A-1 146 767**

**ENGINEERING, vol. 220, no. 4, avril 1980, page 407;**
**"Strecher transit isolator"**

(73) Titulaire: **SOCIETE NOUVELLE D'EXPLOITATION
DE LA CALHENE"S.N.E.L.C.", 1, rue du Petit
Clamart, F-78140 Vélizy (FR)**

(72) Inventeur: **Saint- Martin, Bernard, 6, rue Leclerc,
F-75014 Paris (FR)**
Inventeur: **Picard, Claude, 34, rue Zilina, F-92000
Nanterre (FR)**

(74) Mandataire: **Pottier, Pierre Société BREVATOME,
25, Rue de Ponthieu, F-75008 Paris (FR)**

EP 0 119 916 B1

LIBER, STOCKHOLM 1988

## Description

La présente invention se rapporte à un isolateur local pour la cicatrisation en milieu aseptique d'une plaie ouverte ou fermée, formée sur un membre ou sur toute autre partie du corps d'un malade.

Actuellement, lorsqu'un malade présente une plaie ouverte ou fermée, notamment à la suite d'une intervention chirurgicale, d'une brûlure, d'un accident, etc... il est d'usage d'appliquer sur la plaie un pansement protecteur. Ce pansement est destiné à assurer simultanément la protection de la peau contre la contamination aérienne et la cicatrisation de la plaie. Toutefois, cette technique présente un certain nombre d'inconvénients.

Ainsi, les pansements traditionnels entretiennent le caractère humide et stagnant de l'air qui se trouve en contact avec la plaie. Pour cette raison, et compte tenu du caractère éminemment favorable au développement microbien de la peau, il se produit parfois une macération cutanée. Cette macération augmente sensiblement le temps de cicatrisation et peut conduire dans certains cas à des complications.

Par ailleurs, les pansements conventionnels peuvent être involontairement compressifs et ils peuvent adhérer à la peau. Cela est particulièrement gênant, notamment dans le cas des greffes, et nécessite alors des artifices pour ne pas perturber la prise de la greffe.

L'isolation de la plaie vis-à-vis des autres patients alités dans le même service ou dans le milieu environnant est très théorique lorsqu'on utilise des pansements conventionnels. On est donc souvent conduit, notamment en chirurgie orthopédique, à isoler soigneusement le malade septique dans une partie du service. Ceci exige des équipements coûteux et souvent totalement inefficaces, car de trop nombreux contacts avec l'extérieur détruisent cet isolement. Inversement, cette isolation est fréquemment ressentie par le malade comme une véritable séquestration et elle tend alors à ralentir sa guérison.

Enfin, sachant que le risque de contamination exogène subsiste tant que la cicatrisation de la plaie n'est pas achevée, il existe une certaine discordance entre une intervention chirurgicale menée elle-même en milieu aseptique et la qualité des soins post-opératoires, faute de moyens adaptés. Cette période est d'autant plus critique en cas de brûlure, surtout si l'on est obligé de greffer, ou en cas de cicatrisation dirigée.

Le document DE-A-2 936 256 décrit un isolateur destiné à être utilisé en apesanteur, à bord d'un vaisseau spatial, afin d'éviter que des substances ne se dispersent dans le vaisseau sous l'effet de l'apesanteur. Cet isolateur comprend différentes sections dont certaines présentent des ouvertures pour le passaage des membres d'un patient, ainsi que des gants de manutention et des systèmes d'introduction d'air ou de gaz. Les instruments nécessaires sont introduit avant le décollage par une fermeture à glissière donnant accès à une section d'extrémité.

Le document FR-A-2 188 437 décrit une enceinte étanche permettant d'effectuer une opération sur un patient. La partie à opérer est introduite dans un appendice pourvu d'un champ opératoire que le chirurgien applique sur cette partie avant de l'inciser. Cet appendice est placé dans l'enceinte, qui est pourvue d'un demi-scaphandre par lequel opère le chirurgien.

Aucun de ces documents ne suggère à l'homme du métier d'utiliser un isolateur pour soigner une plaie jusqu'à sa guérison. De plus, les moyens nécessaires aux soins n'y sont pas divulgués.

La présente invention a précisément pour objet un isolateur local destiné à être utilisé à la place des pansements conventionnels et permettant de résoudre les inconvénients de ces pansements qui viennent d'être mentionnés.

A cet effet, et conformément à l'invention, il est proposé un isolateur local comprenant une enceinte étanche, un circuit de ventilation permettant le renouvellement du milieu contenu dans l'enceinte, des moyens comportant au moins une porte pour introduire et évacuer des produits et des matériels dans l'enceinte, des moyens de manipulation permettant d'intervenir à l'intérieur de l'enceinte sans en rompre l'étanchéité, des piquages étanches pour la connexion de tubulures et analogues, et une zone de raccordement format une ouverture pouvant s'adapter sur une partie du corps d'un malade, cet isolateur étant cactérisé en ce qu'il est appliqué à la cicatrisation en milieu aseptique d'une plaie ouverte ou fermée formée sur ladite partie du corps du malade, l'enceinte contenant à cet effet un milieu initialement stérile, au moins un filtre amont sur lequel est branché de façon étanche le circuit de ventilation et au moins un filtre aval étant fixés sur une paroi de l'enceinte, lesdits moyens pour introduire et évacuer des produits et des matériels comportant au moins la porte qui est logée dans une bride de l'enceinte, la porte et la bride étant pourvues de systèmes d'accrochage permettant de les raccorder respectivement à une porte et à une bride d'un isolateur navette contenant un milieu stérile, afin d'introduire et d'évacuer des produits et des matériels dans l'enceinte étanche tout en maintenant le milieu contenu dans cette dernière sous atmosphère stérile, après ouverture de la double porte formée par les portes de l'isolateur local et de l'isolateur navette.

Grâce à un tel isolateur, il est possible d'isoler rigoureusement en milieu aseptique la plaie ou la région opératoire, quel que soit le membre ou la partie du corps sur laquelle elle se trouve, sans pour cela isoler le patient lui-même. Ceci permet d'assurer la continuité de l'aseptie opératoire et la cohabitation de patients septiques et aseptiques dans un même service, sans pour cela bouleverser aucune structure existante, puisque la barrière physique représentée par l'isolateur ne nécessite aucun aménagement particulier du service. De plus, la plaie est à découvert dans l'isolateur, balayée par de l'air stérile, avec éventuellement un apport d'un aérosol aseptique approprié. Ceci permet d'accélérer sensiblement la vitesse des cicatrisations et de contribuer à réduire les temps d'hospitalisation. La suppression des pansements traditionnels évite également des complications post-opératoires très coûteuses en soins, médicaments et temps. En outre, elle diminue les coûts de produits consommables tels que les linges, les pansements conventionnels, etc... Il est aussi possible d'intervenir à tout moment sur la plaie, sans avoir à décoller un pansement. L'isolateur local selon l'invention constitue donc notamment une nouvelle façon de régler le problème des suites opératoires, en protégeant l'opéré de l'environnement, sans le séquestrer pour autant, et tout en facilitant la tâche de l'équipe soignante.

De préférence, et afin de permettre un contrôle efficace de l'avancement de la cicatrisation, l'enceinte étanche est réalisée en un matériau souple et transparent tel que du chlorure de polyvinyle.

Dans un mode de réalisation préféré de l'invention, la zone de raccordement vis-à-vis du malade est interchangeable, de telle sorte que l'isolateur puisse être réutilisé de nombreuses fois.

Dans un mode de réalisation particulier de l'invention, l'enceinte présente une forme allongée et la zone de raccordement présente la forme d'un cône prolongeant l'enceinte à l'une de ses extrémités.

Afin de permettre le traitement d'une plaie située sur n'importe quelle face d'un membre, l'isolateur comporte de préférence des crochets traversant la paroi supérieure de l'enceinte de façon étanche et supportant, à l'intérieur de celle-ci, des étriers à hauteur réglable formant une attelle apte à recevoir ce membre. Un autre crochet peut également être prévu à l'extrémité opposée à la partie de section variable, afin de maintenir le membre en traction lorsque cela st nécessaire. Les moyens pour intervenir à l'intérieur de l'enceinte comprennent de préférence au moins une paire de manchettes avec gants invaginés sur l'enceinte étanche.

On décrira maintenant, à titre d'exemple non limitatif, un mode de réalisation particulier de l'invention en se référant aux dessins annexés dans lesquels :
- la figure 1 est une vue en coupe longitudinale schématique d'un isolateur local selon l'invention destiné à traiter les membres supérieurs ou inférieurs d'un malade, et
- la figure 2 représente une application de l'isolateur de la figure 1 au traitement d'une plaie formée sur la jambe d'un malade.

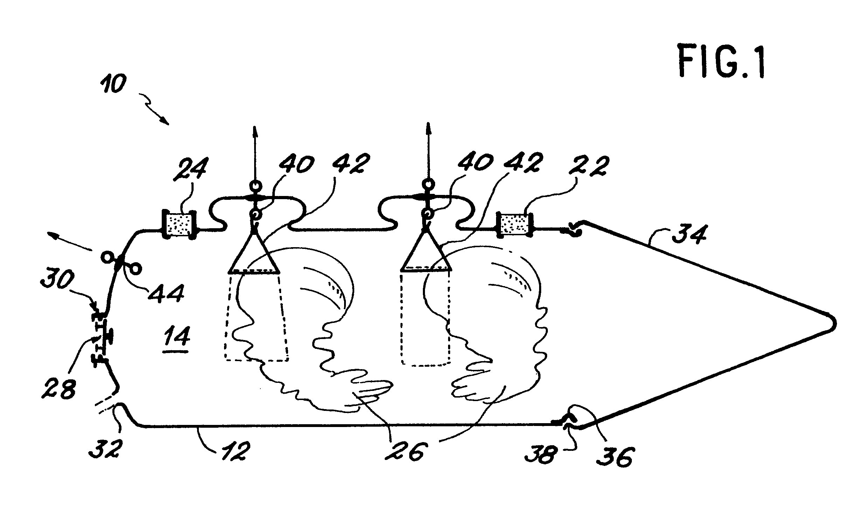

Conformément à l'invention, l'isolateur local 10 représenté sur la figure 1 est destiné à isoler une partie du corps d'un malade présentant une plaie ouverte ou fermée, notamment à la suite d'une intervention chirurgicale, d'un accident, d'une brûlure, etc... De façon plus précise, l'isolateur 10 représenté sur la figure 1 est un isolateur du type "manche" destiné à l'isolement des membres supérieurs ou inférieurs d'un malade. Pour cette raison, un tel isolateur local intéresse plus particulièrement les orthopédistes.

On notera toutefois que l'invention n'est pas limitée à ce type d'isolateur local et peut aussi s'appliquer, par exemple, à des isolateurs de plus grandes dimensions destinés à cicatriser des plaies formées sur d'autres parties du corps d'un malade.

L'isolateur local 10 comprend une enceinte étanche 12, réalisée de préférence en un matériau souple et transparent tel que du chlorure de polyvinyle. Le caractère transparent de l'enceinte permet notamment d'effectuer une surveillance visuelle de la cicatrisation et d'intervenir facilement à l'intérieur de l'enceinte.

L'isolateur de type "manche" représente sur la figure 1 comprend une enceinte ayant la forme d'un cylindre dont les dimensions peuvent être par exemple d'environ 450 mm de diamètre et d'environ 900 mm de long. Le volume clos 14 défini à l'intérieur de l'enceinte étanche 12 contient un milieu aseptique tel que de l'air filtré stérilement, dans lequel on peut introduire, par exemple, un produit cicatrisant lorsque les soins à donner au malade le nécessitent.

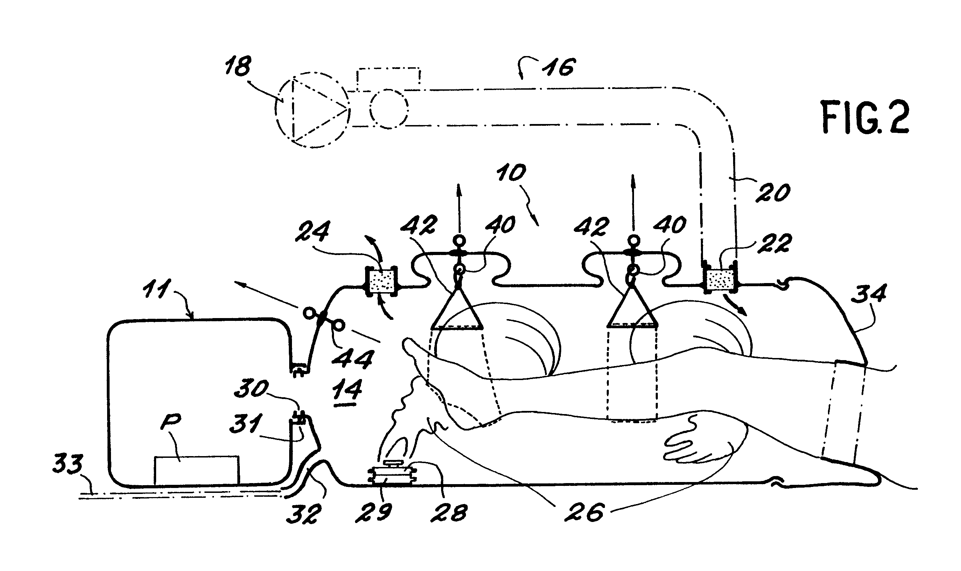

En cours d'utilisation, le milieu contenu dans le volume clos 14 est ventilé et filtré au moyen d'un circuit de ventilation 16 (figure 2) que l'on branche de façon étanche sur un filtre amont 22 fixé sur la paroi cylindrique de l'enceinte 12, à proximité d'une extrémité de celle-ci. Un filtre aval 24, également fixé sur la paroi cylindrique de l'enceinte, à proximité de son extrémité opposée, permet d'évacuer le milieu contenu dans le volume clos 14. Grâce à cet ensemble, on peut assurer le renouvellement régulier de ce milieu tout en supprimant tout rique de contamination croisée entre la chambre du malade et le volume clos 14.

Dans le mode de réalisation représenté sur la figure 2, le circuit 16 est un circuit ouvert qui prélève l'air dans la chambre du malade et le restitue dans cette même chambre au travers du filtre 24. Toutefois, on comprendra que ce circuit peut également être fermé, notamment si le milieu présent dans le volume clos contient des produits autres que de l'air.

Comme on le voit sur la figure 2, le circuit de ventilation 16 comprend un ventilateur 18 actionne par un moteur à vitesse variable ainsi qu'une canalisation 20 reliant ce ventilateur à l'entrée du filtre amont 22.

Le débit du ventilateur 18 nécessaire au renouvellement de l'air contenu dans l'enceinte 12 est suffisamment faible (de 1 à 3 m$^3$/h) pour que l'air puisse être prélevé directement dans la chambre du malade, comme on l'a mentionné précédémment sans que le système de ventilation de celle-ci ne soit perturbé.

En pratique, le ventilateur 18 ainsi que son moteur peuvent être montés sur un support accroché au lit du malade ou fixé au mur, la canalisation 20 étant une canalisation étanche et flexible dont la longueur est suffisante pour permettre au malade de se déplacer lorsque son état l'y autorise.

De préférence, les filtres 22 et 24 sont des filtres de type absolu, qui sont réalisés en papier de verre et montés à l'intérieur d'un carter étanche métallique ou plastique. Chaque filtre présente une efficacité de 99,99 % pour des particules de 0,3 micron. Ces caractéristiques permettent de maintenir le volume clos 14 en atmosphère stérile. Elles permettent aussi de protéger l'enceinte vis-à-vis des contaminants contenus dans l'atmosphère externe.

Conformément à l'invention, l'isolateur local 10 comporte de plus des moyens permettant à une ou plusieurs personnes d'intervenir à l'intérieur du volume clos 14, sans rompre l'étanchéité de l'enceinte 12. Dans la variante de réalisation représentée sur la figure 1, ces moyens consistent en une ou deux paires de manchettes avec gants 26 invaginés sur les côtés de la paroi cylindrique de l'enveloppe 12.

De plus, des moyens doivent être prévus afin d'introduire à volonté des produits ou des appareils à l'intérieur du volume clos 14 et, inversement, d'extraire à volonté des produits et des appareils hors de ce volume. Ces moyens doivent aussi assurer le maintien du volume clos 14 sous atmosphère stérile. A cet effet, on voit sur la figure 1 que l'un des fonds de l'enceinte cylindrique 12 est équipé d'une porte 28 logée dans une bride 30. La porte 28 et la bride 30 sont munies de systèmes d'accrochage, par exemple à baïonnette, permettant de raccorder l'isolateur local 10 à un isolateur navette 11 (figure 2) également équipé d'une porte 29 logée dans une bride 31. Lorsque la bride 31 est raccordée à la bride 30 comme l'illustre la figure 2, les deux portes 28 et 29 également raccordées, peuvent être ouvertes afin de faire communiquer les deux volumes clos définis à l'intérieur des isolateurs 10 et 11, tout en évitant tout risque de contamination croisée.

L'ensemble constitué par les portes 28 et 29 et par les brides 30 et 31 constitue un système de transfert étanche à double porte de type connu.

Grâce à un tel système de double porte, il est possible de relier rapidement l'isolateur local 10 à un isolateur navette 11 dans lequel les matériels et les produits P pouvant être réclamés par le personnel utilisateur sont stockés.

Conformément à l'invention, des piquages étanches tels que 32 sont également prévus dans la paroi de l'enceinte 12, afin de permettre la connexion de tubulures ou de câbles 33 (figure 2) sans rompre l'étanchéité de l'enceinte. Ces piquages étanches peuvent notamment servir au passage de drains, de tubes à vide, de tubes servant à admettre de l'eau filtrée, etc...

Selon une caractéristique essentielle de l'invention, l'enceinte 12 comporte de plus une partie de section variable prévue pour être coupée selon la section désirée, afin d'offrir une ouverture de section correspondant aux mensurations du membre à soigner. Dans la variante de réalisation préférée représentée sur la figure 1, cette partie de l'enceinte 12 est constituée par un cône de latex 34 prolongeant la partie cylindrique de l'enceinte 12 du côté opposé au fond sur lequel est placée la porte 28.

Afin de permettre la réutilisation de l'isolateur local 10 après découpe du cône de latex 34, celui-ci est monté sur l'enceinte 12 de façon démontable.

De façon plus précise, on voit sur la figure 1 que l'extrémité de la partie cylindrique de l'enceinte 12 portant le cône de latex 34 est muni d'un rond de gant 36 comportant une gorge dans laquelle est logé un bourrelet 38 du cône de latex.

Lorsque le membre comportant la plaie à cicatriser est introduit dans l'isolateur local, après découpe du cône de latex 34, il peut soit reposer sur le fond de l'isolateur, sur un champ stérile, soit être placé 5 sur une attelle.

Dans le cas où le membre repose sur le fond de l'isolateur, ce dernier est posé directement sur le lit du malade.

Au contraire, lorsque le membre est placé sur une attelle, il doit être surélevé et même, dans certains cas, être maintenu en traction. A cet effet, on voit sur la figure 1 que l'isolateur local 10 comporte de plus trois crochets traversant l'enceinte 12 de manière étanche. De façon plus précise, deux crochets 40 traversent la cloison supérieure de la partie cylindrique de l'enceinte 12 et soutiennent des étriers 42 à hauteur réglable. Un troisième crochet 44 traverse de façon étanche le fond de l'enceinte 12, au-dessus de la porte 28. Il permet, si besoin est, de mettre en traction le membre soigné dans l'isolateur.

Grâce aux caractéristiques qui viennent d'être décrites, il est possible en plaçant le membre sur les étriers 42 à hauteur réglable, de traiter une plaie se trouvant sur n'importe quelle face de ce membre.

Avant que le cône 34 ne soit découpé afin d'introduire dans l'isolateur local le membre comportant la plaie à cicatriser, il est nécessaire de stériliser l'isolateur local et l'isolateur navette. De plus, on doit introduire dans l'isolateur navette les matériels, linge, produits, etc... qui serviront ultérieurement à effectuer les soins dans l'isolateur local.

Les stérilisations de l'isolateur local et de l'isolateur navette peuvent notamment être réalisées grâce à un appareil de stérilisation automatique introduisant des vapeurs d'acide péracétique suivant un programme défini. En fonction des dimensions des isolateurs locaux et des isolateurs navettes, plusieurs unités peuvent être stérilisées simultanément.

L'isolateur navette 11 est constitué par un isolateur conventionnel, aisément transportable et disposant de deux filtres absolus (non représentés) lui permettant d'être stérilisé comme n'importe quel isolateur.

Lorsque l'isolateur navette contient des éléments souillés, par exemple à la suite d'un raccordement à un autre isolateur local, ces éléments sont emballés dans des sacs en matière plastique souple avant la stérilisation de la navette. Ces sacs, stériles extérieurement, sont alors extraits, puis détruits, par exemple, dans un incinérateur.

L'isolateur navette peut alors venir prendre de nouveaux matériels dans un isolateur de stockage ou "banque" stérile à deux compartiments prévu à cet effet. L'isolateur navette 11 vient se raccorder sur la banque et on y introduit les produits P qui doivent être transférés vers des isolateurs locaux 10. Une fois rempli, l'isolateur navette est transporté jusqu'à la chambre du malade, où il peut être raccordé à l'isolateur local, comme on l'a mentionné précédemment, et lui fournir tous les éléments nécessaires.

Lorsque l'isolateur local et l'isolateur navette ont été ainsi préparés, on découpe suivant la section adéquate correspondant aux mensurations du membre intéressé, le cône de latex 34 obturant l'isolateur local 10, afin d'y introduire ce membre. On a représenté sur la figure 2 le cas où une jambe de malade a été introduite dans l'isolateur 10 et repose sur les étriers 42. On remarquera que le cône de latex est suffisamment élastique pour qu'on puisse y introduire un membre maintenu par un fixateur externe. Bien entendu, la découpe du cône 34 doit être faite de façon à éviter tout serrage excessif du membre.

Lorsque la mise en place d'un isolateur local selon l'invention s'effectue à la suite d'une intervention chirurgicale, deux cas peuvent se présenter.

Dans un premier cas, l'isolateur local est mis en place immédiatement en fin d'intervention. La patient est alors ramené sur son lit avec l'isolateur local et celui-ci est relié au circuit de ventilation 16 lorsque le patient atteint son lit.

Au contraire, si les opérations de brancardage se révèlent difficiles avec l'isolateur local, la mise en place de celui-ci peut être différée et effectuée sur le lit du patient. Dans ce cas, le chirurgien fait un pansement conventionnel qui est retiré dès que le membre est introduit dans l'isolateur ou juste avant.

Lorsque l'isolateur local a été mis en place, la transparence de la paroi de l'enceinte 12 permet, en vision directe, de surveiller la plaie et d'intervenir, grâce aux gants 26 lorsque cela se révèle nécessaire. Comme ou l'a représenté sur la figure 2, l'isolateur navette 11 permet alors, après raccordement des brides 30, 31 et ouverture de la double porte 28, 29 d'introduire des produits et des matériels P dans l'isolateur local 10 et, inversement, d'extraire des produits hors de cet isolateur, tout en maintenant le caractère parfaitement étanche de l'isolateur vis-à-vis de toute contamination croisée.

Il est clair que l'invention qui vient d'être décrite en se référant aux figures 1 et 2 présente de nombreux avantages par rapport aux pansements classiques.

Ainsi, l'isolateur local selon l'invention permet de supprimer toute séquestration du malade septique en autorisant la cicatrisation d'une plaie aseptique dans le milieu ambiant.

De plus, les greffes cutanées sans pansement évoluent favorablement en quelques jours et les cicatrisations dirigées ainsi que la cicatrisation des brûlures se font sans risque de contamination de l'environnement.

De façon générale, l'isolateur local selon l'invention permet d'accélérer sensiblement la vitesse des cicatrisations. Il peut ainsi contribuer à réduire les temps d'hospitalisation, tout en diminuant le coût de produits consommables tels que les pansements conventionnels.

Enfin, l'isolateur local selon l'invention permet de drainer la plaie, de nettoyer la peau et de mobiliser le membre à l'intérieur de l'isolateur, ce qui constitue autant de caractéristiques favorables à la suppression des complications post-opératoires.

Bien entendu, l'invention n'est pas limitée au traitement d'une plaie située sur un membre, mais peut s'appliquer à une plaie située sur toute autre partie du corps. Dans ce cas, le cône consommable 30 est remplacé par une zone de raccordement venant adhérer sur la peau du malade en dégageant la région de la plaie.

## Revendications

1. Isolateur local comprenant une enceinte étanche (12), un circuit de ventilation (16) permettant le renouvellement du milieu contenu dans l'enceinte, des moyens (28, 30) comportant au moins une porte (28) pour introduire et évacuer des produits et des matériels dans l'enceinte, des moyens de manipulation (26) permettant d'intervenir à l'intérieur de l'enceinte sans en rompre l'étanchéité, des piquages étanches (32) pour la conexion de tubulures et analogues, et une zone de raccordement (34) formant une ouverture pouvant s'adapter sur une partie du corps d'un malade, cet isolateur étant caractérisé en ce qu'il est appliqué à la cicatrisation en milieu aseptique d'une plaie ouverte ou fermée, formée sur ladite partie du corps du malade, l'enceinte (12) contenant à cet effet un milieu initialement stérile, au moins un filtre amont (22) sur lequel est branché de façon étanche le circuit de ventilation (16), et au moins un filtre aval (24) étant fixés sur une paroi de l'enceinte, lesdits moyens pour introduire et évacuer des produits et des matériels comportant au moins la porte (28) qui est logée dans une bride (30) de l'enceinte, la porte (28) et la bride (30) étant pourvues de systèmes d'accrochage permettant de les raccorder respectivement à une porte (29) et à une bride (31) d'un isolateur navette (11) contenant un milieu stérile, afin d'introduire et d'évacuer des produits et des matériels (P) dans l'enceinte étanche (12) tout en maintenant le milieu contenu dans cette dernière sous atmosphère stérile, après ouverture de la double porte formée par les portes (28, 29) de l'isolateur local et de l'isolateur navette.

2. Isolateur local selon la revendication 1, caractérisé en ce que l'enceinte étanche (12) est réalisée en un matériau souple et transparent.

3. Isolateur local selon l'une quelconque des revendications 1 et 2, caractérisé en ce que ladite zone de raccordement (34) est interchangeable.

4. Isolateur local selon l'une quelconque des revendications 1 à 3, caractérisé en ce que l'enceinte (12) présente une forme allongée, ladite zone de raccordement (34) présentant la forme d'un cône prolongeant l'enceinte à l'une de ses extrémités.

5. Isolateur local selon l'une quelconque des revendications précédentes, caractérisé en ce qu'il comporte de plus au moins deux crochets (40) traversant la paroi supérieure de l'enceinte (12) de façon étanche et supportant, à l'intérieur de celle-ci, des étriers (42) à hauteur réglable aptes à recevoir ladite partie du corps du malade.

6. Isolateur local selon la revendication 5, prise en combinaison avec la revendication 4, caractérisé en ce qu'il comporte de plus un autre crochet (44) traversant la paroi de l'enceinte opposée à la partie (34) de section variable, afin de maintenir en traction ladite partie du corps du malade.

7. Isolateur local selon l'une quelconque des revendications précédentes, caractérisé en ce que les moyens pour intervenir à l'intérieur de l'enceinte comprennent au moins une paire de manchettes avec gants (26) invaginés sur l'enceinte étanche.

## Patentansprüche

1. Lokalisolationseinrichtung mit einer dichten Kammer (12), einem die Erneuerung des in der Kammer enthaltenen Milieus erlaubenden Ventilationskreis (16), wenigstens eine Tür (28) umfassenden Mitteln (28, 30), um Erzeugnisse und Materialien in die Kammer einzubringen und herauszunehmen, Handhabungsmitteln (26), die einen Eingriff im Inneren der Kammer ohne Aufhebung der Dichte ermöglichen, dichten Rohrstutzen (32) zur Befestigung von Leitungen und ähnlichem und einem Verbindungsbereich (34), der eine Öffnung bildet, welche sich an einen Körperteil des Kranken anpassen kann, wobei diese Isolationseinrichtung dadurch gekennzeichnet ist, daß sie zur Vernarbung einer offenen oder geschlossenen, an dem genannten Körperteil des Kranken gebildeten Wunde in aseptischer Umgebung angewendet wird, die Kammer (12) hierfür ein steriles Ausgangsmilieu, wenigstens stromaufwärts ein Filter (22), an dem dicht der Ventilationskreis (16) angeschlossen ist, und wenigstens stromabwärts ein Filter (24) umfaßt, welches an einer Wand der Kammer befestigt ist, die genannten Mittel zum Einbringen und Herausnehmen von Erzeugnissen und Materialien wenigstens die Tür (28) umfassen, die in einem Flansch (30) der Kammer angeordnet ist, wobei die Tür (28) und der Flansch (30) mit einem Verhakungssystem ausgebildet sind, die ermöglichen, sie mit einer Tür (29) bzw. einem Flansch (31) einer Isolationskammer (11) zu verbinden, die ein steriles Milieu enthält, um Erzeugnisse und Materialien (P) in die dichte Kammer (12) einzubringen und herauszunehmen, wobei das in dieser Letzteren enthaltene Milieu nach dem Öffnen der doppelten Tür, die durch die Türen (28, 29) der Lokalisolationseinrichtung und der Isolationskammer gebildet sind, unter steriler Atmosphäre aufrechterhalten wird.

2. Lokalisolationseinrichtung nach Anspruch 1, dadurch gekennzeichnet, daß die dichte Kammer (12) aus einem weichen und transparenten Material hergestellt ist.

3. Lokalisolationseinrichtung nach irgendeinem der Ansprüche 1 oder 2, dadurch gekennzeichnet, daß der genannte Verbindungsbereich (34) austauschbar ist.

4. Lokalisolationseinrichtung nach irgendeinem der Ansprüche 1 bis 3, dadurch gekennzeichnet, daß die Kammer (12) eine längliche Form aufweist, wobei der Verbindungsbereich (34) die Form eines Kegels aufweist, der die Kammer an einem ihrer Enden verlängert.

5. Lokalisolationseinrichtung nach irgendeinem der vorhergehenden Ansprüche, dadurch gekennzeichnet, daß sie wenigstens zwei Haken (40) aufweist, die die obere Wand der Kammer (12) dicht durchqueren und in ihrem Inneren Bügel (42) mit einstellbarer Höhe halten, die den genannten Körperteil des Kranken aufnehmen können.

6. Lokalisolationseinrichtung nach Anspruch 5 in Kombination mit dem Anspruch 4, dadurch gekennzeichnet, daß sie ferner einen Haken (44) aufweist, der die Wand der Kammer durchquert, die dem Teil (34) mit veränderbarem Querschnitt gegenüberliegt, um den genannten Körperteil des Kranken unter Zug zu halten.

7. Lokalisolationseinrichtung nach irgendeinem der vorhergehenden Ansprüche, dadurch gekennzeichnet, daß die Mittel zum Eingreifen im Inneren der Kammer wenigstens ein Paar Manschetten mit Handschuhen (26) umfassen, die zu der dichten Kammer hin eingestülpt sind.

## Claims

1. Local isolator comprising a tight enclosure (12), a ventilation circuit (16) permitting the replenishment of the medium contained in the enclosure, means (28, 30) having at least one door (28) for introducing and removing products and equipment with respect to the enclosure, handling means (26) making it possible to intervene within the enclosure without breaking the seal, tight orifices (37) for the connection of tubes and the like and a connection zone (34) forming an opening which can be fitted to part of a patient's body, said isolator being characterized in that it is used for healing an open or closed wound on said part of the patient's body in an aseptic medium, the enclosure (12) containing for this purpose an initially sterile medium, at least one upstream filter (22) to which is tightly connected the ventilation circuit (16) and at least one downstream filter (24) is fixed to a wall of the enclosureaid means for introducing and removing products or equipment having at least the door (28) located in a flange (30) of the enclosure, the door (28) and flange (30) being provided with attachment systems permitting their respective connection to a door (29) and flange (31) of a napiform isolator (11) containing a sterile medium in order to introduce and remove the products and equipment (P) with respect to the tight enclosure (12) whilst still maintaining the medium in the latter under a sterile atmosphere after opening the double door formed by the doors (28, 29) of the local isolator and the napiform isolator.

2. Local isolator according to claim 1, characterized in that the tight enclosure (12) is made from a flexible, transparent material.

3. Local isolator according to either of the claims 1 and 2, characterized in that the connection zone (34) is interchangeable.

4. Local isolator according to any one of the claims 1 to 3, characterized in that the enclosure (12) is elongated, the connection zone (34) being in the form of a cone extending one of the ends of the enclosure.

5. Local isolator according to any one of the preceding claims, characterized in that it also has at least two hooks (40) tightly traversing the upper wall of the enclosure (12) and supporting within the same variable height supports (42) able to receive the indicated part of the patient's body.

6. Local isolator according to claim 5, in conjunction with claim 4, characterized in that it also has a further hook (44) passing through the wall of the enclosure opposite to the variable section part (34), in order to maintain said part of the patient's body in traction.

7. Local isolator according to any one of the preceding claims, characterized in that the means for intervening within the enclosure comprise at least one pair of cuffs with gloves (26) invaginated onto the tight enclosure.

FIG.1

FIG.2